# EUROPÄISCHE PATENTANMELDUNG

(11) **EP 4 556 462 A1**
(43) Veröffentlichungstag der Anmeldung: **21.05.2025**
(21) Anmeldenummer: 23210489.3
(22) Anmeldetag: 17.11.2023
(51) Int. Cl.: C07C 273/18, C07C 275/64

(54) **VERFAHREN ZUR HERSTELLUNG VON SUBSTITUIERTEN PHENYLHARNSTOFFDERIVATEN**

(71) Anmelder: LANXESS Deutschland GmbH, 50569 Köln (DE)
(72) Erfinder: Prinz, Thomas, 51371 Leverkusen (DE); Suberg, Marcus, 41352 Korschenbroich (DE); Halle, Olaf, 51061 Köln (DE); Leiberich, Ricarda, 63150 Heusenstamm (DE); Müller, Mauritio, 83022 Rosenheim (DE); Böger, Uwe, 51375 Leverkusen (DE)

(57) **Zusammenfassung**

Die vorliegende Erfindung betrifft ein neues Verfahren zur Herstellung von substituierten Phenylharnstoffderivaten, insbesondere 3-(3,4-dichlorphenyl)-1,1-dimethylharnstoff.

## Beschreibung

Die vorliegende Erfindung betrifft ein neues Verfahren zur Herstellung von substituierten Phenylharnstoffderivaten, insbesondere 3-(3,4-Dichlorphenyl)-1,1-dimethylharnstoff.

### Stand der Technik

Phenylharnstoffe sind eine Klasse von selektiv wirksamen Herbiziden; so ist 3-(3,4-Dichlorphenyl)-1,1-dimethylharnstoff (DCMU) ein Herbizid, das die Photosynthese von Pflanzen hemmt. Es wird zur völligen Beseitigung von Pflanzen verwendet (Breitbandherbizid) ebenso wie zum Schutz von Holz und Mauerwerk und als Beschichtungsmittel.

Im Stand der Technik sind verschiedene Ansätze zur Herstellung von Phenylharnstoffderivaten bekannt, insbesondere die Herstellung von 3-(3,4-Dichlorphenyl)-1,1-dimethylharnstoff (DCMU) aus 3,4-Dichlorphenylisocyanat und Dimethylamin, die entweder in Lösungsmitteln oder in der Schmelze durchgeführt werden.

Bei den in DE-A-2206167 und DD-A-201140 beschriebenen Schmelzeverfahren, die als Semi-Batch-Prozesse in gerührten Reaktoren durchgeführt werden, muss aufgrund der hohen Exothermie der Reaktion durch geeignete technische Maßnahmen eine ausreichende Wärmeabfuhr und damit Temperaturkontrolle sichergestellt werden. Hierfür werden beide Edukte dosierkontrolliert zugegeben, was durch die damit verbundenen langen Verweilzeiten bei Temperaturen oberhalb des Schmelzpunkts des Produkts zur Bildung unerwünschter Nebenprodukte führt. Bei durch unzureichende Durchmischung hervorgerufenem lokalen Aminmangel wird das Isocyanat nicht hinreichend schnell umgesetzt, was zur thermischen Zersetzung oder unerwünschter Dimerisierung und Trimerisierung (Tris-(3,4-dichlorphenyl)-1,3,5-triazin-2,4,6-trion) des Isocyanats führt.

Bei den Lösungsmittelverfahren wird in der Regel auf ein mit Wasser nicht mischbares Lösungsmittel, wie Benzol, Toluol oder Chlorbenzol, oft in Kombination mit einer wässrigen Lösung des Amins zurückgegriffen, siehe beispielsweise DE-A 3245679 oder CN-A- 103539704, was den Nachteil hat, dass dieses Lösungsmittel nach der Umsetzung aufwändig entfernt und zur Wiederverwendung aufbereitet werden muss. Die Verwendung wässriger Aminlösungen führt zudem zur Hydrolyse des Isocyanats, was wiederum die Bildung unerwünschter Biphenylharnstoffe bedingt.

Aus HU178312 ist ebenfalls ein Lösungsmittelverfahren bekannt, bei dem Wasser als Lösungsmittel dient und die Umsetzung von 3,4-Dichlorphenylisocyanat mit Dimethylamin als Konti-Verfahren beschrieben ist. Hierbei wird das Amin zur Minimierung der Bildung unerwünschter Nebenprodukte im hohen molaren Überschuss eingesetzt. Die Anwesenheit von Wasser führt aber auch hier zur Bildung signifikanter Mengen von Biphenylharnstoffen (1,3-Bis-(3,4-dichlorphenyl)harnstoff), die mit dem Zielprodukt ausfallen und ohne weitere Aufreinigungsschritte nicht aus dem Produkt entfernt werden können. Zudem ist das Abwasser intensiv zu reinigen, da die vorgenannten Verbindungen stark gewässergefährdend sind.

### Aufgabe der vorliegenden Erfindung

Die Aufgabe der vorliegenden Erfindung war es daher, ein effizientes Verfahren zur Herstellung von substituierten Phenylharnstoffderivaten, insbesondere von 3-(3,4-Dichlorphenyl)-1,1-dimethylharnstoff, bereitzustellen, welches die Probleme des Standes der Technik, wie die aufwändige Abtrennung und Reinigung des Lösungsmittels und den Anfall von Biphenylharnstoffen (1,3-Bis-(3,4-dichlorphenyl)harnstoff) oder die Dimerisierung bzw. Trimerisierung des Isocyanats nicht aufweist.

### Lösung der Aufgabe

Überraschenderweise wurde nun gefunden, dass die der Erfindung zugrunde liegende Aufgabe dadurch gelöst wird, dass die Umsetzung von substituierten Phenylharnstoffderivaten mit Dimethylamin und/oder N,O-Dimethylhydroxylamin in einem Reaktor durchgeführt wird, der ein Verhältnis von Oberfläche zu Volumen von 100 bis 5000m²/m³ aufweist.

### Gegenstand der Erfindung

Gegenstand der Erfindung ist somit ein Verfahren zur Herstellung von substituierten Phenylharnstoffderivaten der Formel (I) 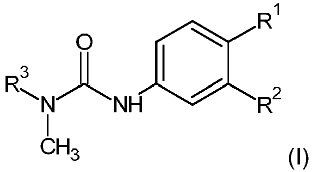
mit R¹ = H, Methyl, F, Cl oder Br,
R² = H, F, Cl, Br, vorzugsweise Cl, oder C₁ - C₃-Alkyl, oder einfach oder mehrfach mit F, Cl oder Br substituiertes C₁ - C₃-Alkyl, vorzugsweise CF₃,
wobei R¹ und R² nicht gleichzeitig H bedeuten
   und
R³ = CH₃ oder OCH₃,
durch die Umsetzung von Verbindungen der Formel (II) 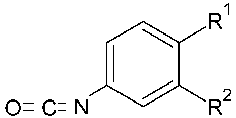
mit einem Amin der Formel (III) 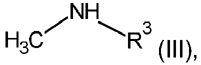
wobei R¹, R² und R³ die vorgenannte Bedeutung haben, und wobei die Umsetzung in einem Reaktor durchgeführt wird, der ein Verhältnis von Oberfläche zu Volumen von 100 bis 5000m²/m³ aufweist.

Besonders bevorzugt handelt es sich bei den substituierten Phenylharnstoffderivaten der Formel (I) um die nachstehend genannten Verbindungen: 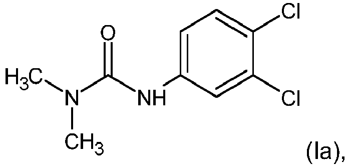 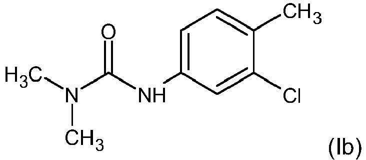 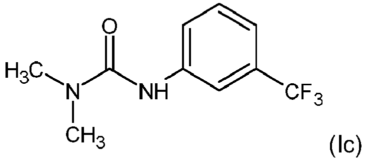 und/oder

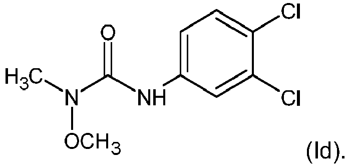

Die Verbindung der Formel (la) 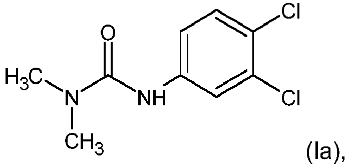 wird vorzugsweise hergestellt über die Umsetzung von 3,4-Dichlorphenylisocyanat mit Dimethylamin.

Die Verbindung der Formel (Ib) 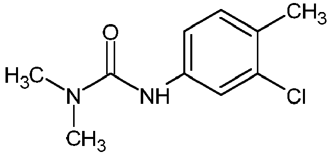 wird dabei vorzugsweise hergestellt über die Umsetzung von 3-Chlor-4-methylphenylisocyanat mit Dimethylamin.

Die Verbindung der Formel (Ic) 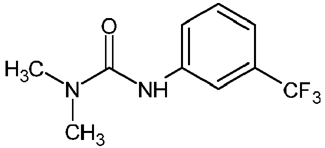 wird dabei vorzugsweise hergestellt über die Umsetzung von 3-Trifluormethylphenylisocyanat mit Dimethylamin.

Die Verbindung der Formel (Id) 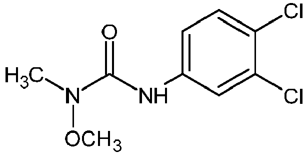 wird dabei vorzugsweise hergestellt über die Umsetzung von 3,4-Dichlorphenylisocyanat mit einem Amin der Formel (III) 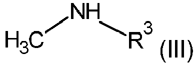 mit R³ = OCH₃.

In einer bevorzugten Ausführungsform der Erfindung beträgt das Verhältnis von Oberfläche zu Volumen 500 bis 3500m²/m³, besonders bevorzugt 650 bis 2000m²/m³.

Unter Reaktor im Sinne der Erfindung wird vorzugsweise ein rohrförmiger Reaktor verstanden. Bevorzugt sind Rohrreaktoren oder Rohrbündelreaktoren oder Mikroreaktoren. Der bevorzugte rohrförmige Reaktor wird vorzugsweise von einem Kühlmedium, wie beispielsweise Thermalöl, umflossen.

In einer besonders bevorzugten Ausführungsform der Erfindung weisen der Reaktor bzw. bei einem Rohrbündelreaktor die einzelnen Rohre des Reaktors einen Durchmesser von 0,8 mm bis 40 mm, besonders bevorzugt 2 bis 6 mm auf. Die Reaktorlänge beträgt vorzugsweise 0,4 bis 3 m.

In einer weiteren bevorzugten Ausführungsform der Erfindung sind die Reaktoren mit statischen Mischern ausgestattet.

Ein statischer Mischer oder Statikmischer ist eine Vorrichtung zum Mischen von Fluiden, in der vorzugsweise allein die Strömungsbewegung die Vermischung bewirkt und der nicht über bewegte Elemente verfügt. Er besteht vorzugsweise aus strömungsbeeinflussenden Elementen in einem Rohr, bevorzugt aus einem oder mehreren unterschiedlich angeordneten Elementen, die den Stoffstrom abwechselnd teilen und wieder zusammenführen, wodurch die Vermischung erreicht wird.

In einer weiteren bevorzugten Ausführungsform der Erfindung beträgt die Verweilzeit im Reaktor 5-120 Sekunden, vorzugsweise 20-60 Sekunden.

Des Weiteren ist bevorzugt, dass der Anteil von Wasser und/oder organischen Lösungsmitteln, wie vorzugsweise Toluol, 0 - 0,2 Gew.%, bezogen auf die Gesamtmischung, beträgt.

Zudem ist es bevorzugt, dass die Herstellung kontinuierlich durchgeführt wird, d.h. beide Edukte kontinuierlich im gewünschten molaren Verhältnis dem Reaktor zugeführt werden.

In einer bevorzugten Ausführungsform der Erfindung beträgt das molare Verhältnis von Amin der Formel (III) zum substituierten Phenylisocyanat der Formel (II) 2 : 1 bis 10 : 1, vorzugsweise 3 : 1 bis 6 : 1. In dem erfindungsgemäßen Verfahren wird das substituierte Phenylisocyanat vorzugsweise über Düsen in das vorzugsweise im Überschuss vorliegende Amin der Formel (III) eingeleitet.

In einer weiteren bevorzugten Ausführungsform der Erfindung erfolgt das erfindungsgemäße Verfahren bei einem Druck von 1 bis 100 bar, vorzugsweise 5 bis 60 bar, besonders bevorzugt 10 bis 55 bar.

In einer weiteren bevorzugten Ausführungsform der Erfindung wird das erfindungsgemäße Verfahren bei einer Temperatur von 1 bis 30°C über dem Schmelzpunkt des substituierten Phenylharnstoffderivates der Formel (I), vorzugsweise bei einem Druck von 1 bis 100 bar, durchgeführt, im Falle von 3-(3,4-Dichlorphenyl)-1,1-dimethylharnstoff also bei 160 bis 190°C, vorzugsweise bei einem Druck von 1 bis 100 bar.

Das bei dem erfindungsgemäßen Verfahren vorzugsweise im molaren Überschuss eingesetzte Amin der Formel (III) wird nach der Umsetzung mit dem substituierten Phenylisocyanat der Formel (II), vorzugsweise dem 3,4-Dichlorphenylisocyanat, vom Endprodukt, dem substituierten Phenylharnstoffderivat der Formel (I), abgetrennt und das überschüssige Amin vorzugsweise wieder in den Reaktor zurückgeführt. Dies kann beispielsweise durch Rückführung in den Aminvorratsbehälter erfolgen aus dem dann die Zudosierung in den Reaktor erfolgt.

### Bevorzugte Ausführungsform des Verfahrens:

In einer bevorzugten Ausführungsform der Erfindung erfolgt das erfindungsgemäße Verfahren am Beispiel der Herstellung von 3-(3,4-Dichlorphenyl)-1,1-dimethylharnstoff wie folgt:
In einen öltemperierten Rohrreaktor mit einem Verhältnis von Oberfläche zu Volumen von 1000m²/m³ und mit einem Durchmesser von 4mm, ausgestattet mit statischen Mischern, wird auf 120 bis 150°C vortemperiertes Dimethylamin eingeleitet. Über eine am Anfang des Rohrreaktors eingebaute Düse wird auf 100-120°C vorgeheiztes 3,4-Dichlorphenylisocyanat so eingeleitet, dass ein molares Verhältnis von Dimethylamin zu 3,4-Dichlorphenylisocyanat von 3 : 1 bis 10:1 eingehalten wird. Die Vermischung von Dimethylamin mit 3,4-Dichlorphenylisocyanat erfolgt idealerweise mit einem statischen Mischer. Der Druck liegt vorzugsweise bei 20 - 50 bar, die Reaktortemperatur bei 160 bis 185°C und die Verweilzeit des Reaktionsmediums bei ca. 30s. Am Ende des Rohrreaktors erfolgt dann eine Entspannung auf Umgebungsdruck, gefolgt von der Trennung des 3-(3,4-Dichlorphenyl)-1,1-dimethylharnstoffs vom im Überschuss eingesetzten Dimethylamin durch Entgasung.

Für die anderen substituierten Phenylharnstoffderivate gemäß Formel (I) erfolgt das erfindungsgemäße Verfahren analog. Die Reaktortemperaturen werden entsprechend des Schmelzpunkts des substituierten Phenylharnstoffderivates der Formel (I) angepasst und liegen vorzugsweise bei einer Temperatur von 1 bis 30°C über dem Schmelzpunkt des substituierten Phenylharnstoffderivates der Formel (I).

Im Folgenden wird die Erfindung an Beispielen und Vergleichsbeispielen beschrieben. Die folgenden Beispiele sind jedoch nur bevorzugte Beispiele für die vorliegende Erfindung und die vorliegende Erfindung ist nicht auf die folgenden Beispiele beschränkt.

### Beispiele

### Herstellung von 3-(3,4-Dichlorphenyl)-1,1-dimethylharnstoff (Schmelzpunkt: 158-159°C)

### Beispiel 1 (erfindungsgemäß):

In einen öltemperierten Rohrreaktor mit einem Oberflächen/Volumen-Verhältnis von 1000m²/m³ (Durchmesser 4mm), ausgestattet mit statischen Mischern, wurden 262g/h auf 120°C vortemperiertes Dimethylamin kontinuierlich eingeleitet. Über eine unmittelbar am Anfang des Reaktionsrohrs eingebaute Düse wurden kontinuierlich 273g/h auf 130°C vorgeheiztes 3,4-Dichlorphenylisocyanat in das Dimethylamin eingedüst und mithilfe statischer Mischer vermischt. Das molare Verhältnis von Dimethylamin zu 3,4-Dichlorphenylisocyanat betrug 4:1. Die maximale im Rohrreaktor auftretende Temperatur lag bei ca. 185°C. Der Druck lag bei 55bar, die Verweilzeit des Reaktionsmediums lag bei ca. 60s. Am Ende des Reaktionsrohres erfolgte eine Entspannung auf Umgebungsdruck, gefolgt von der Entgasung des Reaktionsprodukts vom im Überschuss eingesetzten Amin.

Der Umsatz lag bei >99,998% bezogen auf das eingesetzte Isocyanat, die Reinheit des gewonnenen Produkts lag bei >99,7%.

Der Gehalt des infolge von Hydrolyse und thermischer Zersetzung des Isocyanats entstehenden Nebenprodukts 1,3-Bis-(3,4-dichlorphenyl)harnstoff lag bei unter 50ppm.

Unerwünschte Dimerisierung und Trimerisierung des Isocyanats sowie kritische Nebenprodukte wie 3,4,3',4'-Tetrachlorazobenzol und 3,4,3',4'-Tetrachlorazoxybenzol waren nicht nachweisbar.

### Beispiel 2 (erfindungsgemäß):

Das Verfahren wurde gemäß Beispiel 1 bei gleichem molaren Verhältnis von Dimethylamin zu 3,4-Dichlorphenylisocyanat von 4:1 wiederholt, mit dem Unterschied, dass die Verweilzeit ca. 30s, der Druck 55 bar und die maximale Temperatur 189 °C betrugen.

Der Umsatz lag bei >99,997% bezogen auf das eingesetzte Isocyanat, die Reinheit bei >99,7%.

Der Anteil an 1,3-Bis-(3,4-dichlorphenyl)harnstoff war <50ppm, eine unerwünschte Dimerisierung und Trimerisierung des Isocyanats trat nicht auf und 3,4,3',4'-Tetrachlorazobenzol und 3,4,3',4'-Tetrachlorazoxybenzol waren nicht nachweisbar.

### Beispiel 3 (erfindungsgemäß):

Das Verfahren wurde gemäß Beispiel 1 wiederholt, mit dem Unterschied, dass der Druck auf 23bar reduziert wurde.

Der Umsatz lag bei >99,996%, die Reinheit des 3-(3,4-Dichlorphenyl)-1,1-dimethylharnstoffs bei >99,6%.

Der Anteil an 1,3-Bis-(3,4-dichlorphenyl)harnstoff war <50ppm, eine unerwünschte Dimerisierung und Trimerisierung des Isocyanats trat nicht auf und 3,4,3',4'-Tetrachlorazobenzol und 3,4,3',4'-Tetrachlorazoxybenzol waren nicht nachweisbar.

### Beispiel 4 (Vergleichsbeispiel) nach HU178312

In einen kontinuierlich betriebenen Rührkesselreaktor mit einem Oberflächen/VolumenVerhältnis in der Größenordnung von ca. 50 m²/m³, ausgestattet mit einem Intensivmischer, wurde eine im Kreis geführte 25%ige wässrige Dimethylaminlösung eingeleitet, sodass die Verweilzeit der Reaktionsmedien im Bereich von ca. 40s lag. Zusätzlich wurden als Feed eine 58%ige wässrige Dimethylaminlösung und geschmolzenes 3,4-Dichlorpenylisocyanat im stöchiometrischen Verhältnis von 1:1 zugegeben. Das molare Verhältnis des gesamten zugeführten Dimethylamins zum Isocyanat betrug ca. 23:1. Die Reaktionstemperatur betrug 40-45°C.

Das innerhalb einer Stunde im nachgeschalteten Filter gesammelte Produkt wurde getrocknet. Der Umsatz lag bei 98% bezogen auf das eingesetzte Isocyanat, die Reinheit des gewonnenen Produkts bei 98,5%.

Der Anteil an 1,3-Bis-(3,4-dichlorphenyl)harnstoff betrug > 1%, 3,4,3',4'-Tetrachlorazobenzol und 3,4,3',4'-Tetrachlorazoxybenzol waren nicht nachweisbar.

### Resümee:

Es zeigte sich, dass mit dem erfindungsgemäßen Verfahren substituierte Phenylharnstoffderivate der Formel (I) in hoher Ausbeute und Reinheit erhalten wurden und dass der Anteil an 1,3-Bis-(3,4-dichlorphenyl)harnstoff <50ppm war und 3,4,3',4'-Tetrachlorazobenzol und 3,4,3',4'-Tetrachlorazoxybenzol nicht nachweisbar sind.

## Patentansprüche

1. Verfahren zur Herstellung von substituierten Phenylharnstoffderivaten der Formel (I) 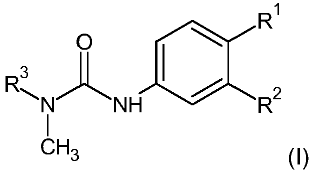
mit R¹ = H, Methyl, F, Cl oder Br,
R² = H, F, Cl, Br, vorzugsweise Cl, oder C₁ - C₃-Alkyl, oder einfach oder mehrfach mit F, Cl oder Br substituiertes C₁ - C₃-Alkyl, vorzugsweise CF₃,
wobei R¹ und R² nicht gleichzeitig H bedeuten
und
R³ = CH₃ oder OCH₃,
durch die Umsetzung von Verbindungen der Formel (II) 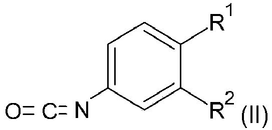
mit einem Amin der Formel (III) 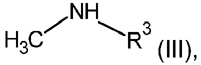 wobei R¹, R² und R³ die vorgenannte Bedeutung haben, **dadurch gekennzeichnet, dass** die Umsetzung in einem Reaktor durchgeführt wird, der ein Verhältnis von Oberfläche zu Volumen von 100 bis 5000m²/m³, bevorzugt 500 bis 3500m²/m³, besonders bevorzugt 650 bis 2000m²/m, aufweist.

2. Verfahren nach Anspruch 1, **dadurch gekennzeichnet, dass** es sich bei den Verbindungen der Formel (I) um 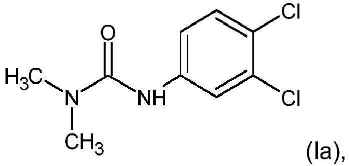 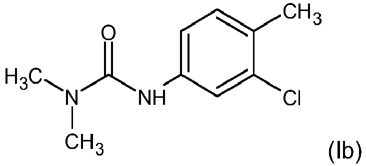 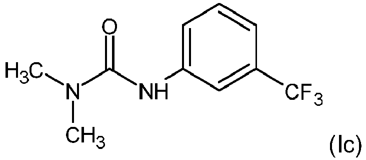 und/oder 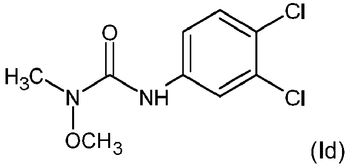 handelt.

3. Verfahren nach Anspruch 1 bis 2, **dadurch gekennzeichnet, dass** es sich bei dem Reaktor um einen Rohrreaktor handelt.

4. Verfahren nach wenigstens einem der Ansprüche 1 bis 3, **dadurch gekennzeichnet, dass** der Rohrreaktor einen Durchmesser von 0,8 mm bis 40 mm aufweist.

5. Verfahren nach wenigstens einem der Ansprüche 1 bis 4, **dadurch gekennzeichnet, dass** es sich bei dem Rohrreaktor um einen Rohrbündelrektor oder einen Mikroreaktor handelt.

6. Verfahren nach wenigstens einem der Ansprüche 1 bis 5, **dadurch gekennzeichnet, dass** die Reaktion kontinuierlich durchgeführt wird.

7. Verfahren nach wenigstens einem der Ansprüche 1 bis 6, **dadurch gekennzeichnet, dass** das Amin nach Formel (III) und das substituierte Phenylisocyanat nach Formel (II) im molaren Verhältnis von 2 : 1 bis 10:1, vorzugsweise 3 : 1 bis 6 : 1, eingesetzt werden.

8. Verfahren nach wenigstens einem der Ansprüche 1 bis 7, **dadurch gekennzeichnet, dass** die Umsetzung bei einem Druck von 1 bis 100 bar, vorzugsweise 5 bis 60 bar, besonders bevorzugt 10 bis 55 bar durchgeführt wird.

9. Verfahren nach wenigstens einem der Ansprüche 1 bis 8, **dadurch gekennzeichnet, dass** die Umsetzung bei einer Temperatur von 1 bis 30°C über dem Schmelzpunkt des substituierten Phenylharnstoffderivates der Formel (I) bei einem Druck von 1 bis 100 bar, vorzugsweise 5 bis 60 bar, besonders bevorzugt 10 - 55 bar durchgeführt wird.

10. Verfahren nach Anspruch 7, **dadurch gekennzeichnet, dass** das überschüssige Amin der Formel (III) nach der Umsetzung in den Reaktor zurückgeführt wird.

11. Verfahren nach wenigstens einem der Ansprüche 1 bis 10, **dadurch gekennzeichnet, dass** die Verweilzeit im Reaktor 5-120 Sekunden beträgt.
